Europäisches Patentamt

European Patent Office

Office européen des brevets

(11) Veröffentlichungsnummer: **0 583 628 A1**

# (12) EUROPÄISCHE PATENTANMELDUNG

(21) Anmeldenummer: **93111508.3**

(22) Anmeldetag: **17.07.93**

(51) Int. Cl.5: **C07C 305/04**, C07C 303/24, C07C 303/44

(30) Priorität: **17.08.92 DE 4227210**

(43) Veröffentlichungstag der Anmeldung:
**23.02.94 Patentblatt 94/08**

(84) Benannte Vertragsstaaten:
**BE DE ES FR GB IT NL**

(71) Anmelder: **CHEMISCHE WERKE HÜLS AG**
**- RSP Patente / PB 15 -**
**D-45764 Marl(DE)**

(72) Erfinder: **Stehr, Michael, Dr.**
**Heimgarten 17**
**D-4650 Gelsenkirchen(DE)**
Erfinder: **Otte, Adelbert**
**Stübbenfeldstrasse 15**
**D-4370 Marl(DE)**
Erfinder: **Glaser, Helmut, Dr.**
**Pierenkemper 59**
**D-4650 Gelsenkirchen(DE)**
Erfinder: **Ratajczak, Hans-Josef, Dr.**
**Leunaer Strasse 13**
**D-4370 Marl(DE)**
Erfinder: **Schulze, Klaus, Dr.**
**Zum Seeblick 20**
**D-4358 Haltern(DE)**

(54) **Verfahren zur Herstellung von Alkylsulfat-Pulvern mit hohem Schüttgewicht.**

(57) Die Erfindung betrifft ein Verfahren zur Herstellung von Alkylsulfat-Pulvern mit einem Schüttgewicht von mindestens 300 g/l durch Sprühtrocknung einer wäßrigen Lösung von Alkylsulfat mit einem Feststoffgehalt von 10 bis 60 Gew.-% in einem Sprühturm, in dessen Kopfteil die Flüssigkeit durch einen Druck von $10^6$ bis $10^7$ Pa durch Einstoffdüsen versprüht und der so entstehende Sprühnebel im Gleichstrom mit heißem Inertgas getrocknet wird.

EP 0 583 628 A1

Die Erfindung betrifft ein Verfahren zur Herstellung von Alkylsulfat-Pulvern mit einem Schüttgewicht von mindestens 300 g/l durch Sprühtrocknung einer wäßrigen Lösung oder Dispersion von Alkylsulfaten mit einem Feststoffgehalt von 10 bis 60 Gew.-% in einem Sprühturm, in dessen Kopfteil die Flüssigkeit durch Druck durch Einstoffdüsen versprüht und der so entstehende Sprühnebel im Gleichstrom mit heißem Inertgas getrocknet wird. Mit Alkylsulfaten werden im folgenden sowohl Alkylsulfate mit einheitlicher Kohlenstoffzahl als auch Mischungen von Alkylsulfaten mit unterschiedlicher Kohlenstoffzahl in der Alkylkette bezeichnet.

Die Alkalisalze von Monoalkylschwefelsäurehalbestern - nachfolgend Alkylsulfate genannt -, deren Alkylgruppe zwischen 8 und 22 Kohlenstoffatome enthält, finden als (anaerob abbaubare) Anionentenside zunehmend größeres Interesse.

Alkylsulfate werden im technischen Maßstab durch Umsetzung von Alkoholen mit Schwefeltrioxid und anschließende Neutralisation des primär gebildeten, chemisch labilen Schwefelsäurehalbesters mit geeigneten wäßrigen Basen, wie z. B. Natronlauge oder wäßriger Natriumcarbonatlösung, gewonnen. Die Alkylsulfate fallen als wäßrige Lösungen, Dispersionen oder Pasten an. Diese Darreichungsformen erschweren häufig die Weiterverarbeitung und sind für bestimmte Anwendungen ungeeignet; besonders die Anwesenheit von Wasser wird als störend empfunden und verteuert den Transport.

Die Entfernung von Wasser kann nun auf die vielfältigste Art und Weise geschehen, nur gilt es zu beachten, daß der gewählte Prozeß zu einem Feststoff fuhrt, der gut weiterverarbeitet werden kann.

Besonders vorteilhaft ist es, Alkylsulfat-Pulver herzustellen, da diese im Gegensatz beispielsweise zu Granulaten oder Schuppen direkt mit anderen Pulvern zu Endformulierungen abgemischt werden können und zudem eine schnelle Wasserlöslichkeit besitzen. Um wäßrige Lösungen von Alkylsulfaten in Pulver zu überführen, sind prinzipiell mehrere Verfahren denkbar; welches Verfahren zur Anwendung kommt, hängt sowohl von den physikalischen und chemischen Eigenschaften der zu trocknenden Alkylsulfat-Lösung als auch von den gewünschten Pulvereigenschaften ab.

Ein an sich geeignetes Verfahren ist die Sprühtrocknung, jedoch lehrt die JP 54 10 6428, daß Alkylsulfat-Slurries mindestens einen Feststoffgehalt von 60 bis 80 % haben müssen, um Pulver mit Schüttgewichten oberhalb von 200 g/l herstellen zu können, was für die weitere Handhabung immer noch unbefriedigend niedrig ist. Slurries mit einem derartig hohen Tensidgehalt sind nach der Lehre der EP 0 084 154 in der Technik nur zu handhaben, wenn besondere Maßnahmen wie z. B. eine Additivierung durchgeführt werden. Die zu diesem Zweck einsetzbaren Stoffe haben jedoch gewisse Nachteile. Sie können z. B. die Einsetzbarkeit der Pulver in einer Endformulierung beeinträchtigen oder durch einen niedrigen Schmelzpunkt die Versprühung selbst erschweren, da sie ein Verkleben der Pulver fördern.

Es bestand daher die Aufgabe, Alkylsulfat-Pulver mit einem Schüttgewicht von mindestens 300 g/l ausgehend von fließfähigen, wäßrigen Alkylsulfat-Lösungen oder -Dispersionen ohne derartige Additive herzustellen.

Die Aufgabe wird erfindungsgemäß durch ein Verfahren gelöst, bei dem man wäßrige Lösungen oder Dispersionen von Alkylsulfaten mit einem Feststoffgehalt von 10 bis 60 % in den Kopfteil eines Sprühturmes durch Einstoffdüsen versprüht und den so entstehenden Sprühnebel mit heißem Inertgas im Gleichstrom trocknet.

Die wäßrige Lösung oder Dispersion wird mittels handelsüblicher Druckdüsen zwischen $10^6$ und $10^7$ Pa in den Sprühturm gedüst. Als Speise für die Verdüsung kommen alle Lösungen von üblichen sulfatierten Fettalkoholschnitten in Frage, sofern ihre Viskosität ein Pumpen und Verdüsen zuläßt.

Als Inertgas wird aus ökonomischen Gründen Stickstoff bevorzugt.

Gegenstand der Erfindung ist mithin ein Verfahren zur Herstellung von Alkylsulfat-Pulvern, dadurch gekennzeichnet, daß man die wäßrige Lösung oder Dispersion von Alkylsulfaten in den Kopfteil eines Sprühturmes durch Einstoffdüsen unter Druck versprüht und den so entstehenden Sprühnebel im Gleichstrom mit heißem Inertgas zu einem Pulver mit einem Schüttgewicht von mindestens 300 g/l trocknet.

Folgende Beispiele zeigen die nach der vorliegenden Erfindung erzielbaren deutlichen Erhöhungen des Schüttgewichtes.

Beispiel 1 und Vergleichsbeispiel 1

Die Trocknung einer handelsüblichen 30%igen wäßrigen Lösung von Kokosfettalkoholsulfat ($C_{12}$ - $C_{14}$) in einem mit Stickstoff betriebenen Gleichstromturm erbrachte Pulver mit in der Tabelle 1 beschriebenen Eigenschaften.

| Tabelle 1 | Beispiel erfindungsgemäß | Vergleichsbeispiel |
|---|---|---|
| Sprühbedingungen | | |
| Düse | Einstoff-Hohlkegeldüse des Types DELAVAN 2,4 P7 | Zweistoffdüse DELA-VAN |
| Speisedruck | $10^6$ Pa (10 bar) | $3 \cdot 10^5$ Pa (3 bar) |
| Temperatur des Gases für die Verdüsung | entfällt | 60 °C |
| Druck des Gases für die Verdüsung | entfällt | $5 \cdot 10^5$ Pa |
| Gaseintrittstemperatur | 155 °C | 155 °C |
| Gasaustrittstemperatur | 115 °C | 115 °C |
| Inertgasmenge (Kreis-gas) | 30 t $N_2$ | 30 t $N_2$ |
| Pulverdaten | | |
| Schüttgewicht | 360 g/l | 100 g/l |
| Wassergehalt nach Karl Fischer | 1,7 % | 1,2 % |

Durch das erfindungsgemäße Verfahren wird ein Alkylsulfat-Pulver mit einem Schüttgewicht von 360 g/l erhalten, während die auf herkömmliche Weise mit einer Zweistoffdüse versprühte Lösung nur ein Schüttgewicht des Alkylsulfats von 100 g/l ergibt.

Beispiel 2 und Vergleichsbeispiel 2

Die Trocknung einer handelsüblichen 30%igen wäßrigen Lösung von $C_{12}$ - $C_{18}$-Fettalkoholsulfat in einem mit Stickstoff betriebenen Gleichstromturm erbrachte Pulver mit in der Tabelle 2 beschriebenen Eigenschaften.

3

| Tabelle 2 | Beispiel erfindungsgemäß | Vergleichsbeispiel |
|---|---|---|
| Sprühbedingungen | | |
| Düse | Einstoff-Hohlkegeldüse des Types DELAVAN 2,4 P7 | Zweistoffdüse DELAVAN |
| Speisedruck | $2 \cdot 10^6$ Pa (20 bar) | $3 \cdot 10^5$ Pa (3 bar) |
| Temperatur des Gases für die Verdüsung | entfällt | 60 °C |
| Druck des Gases für die Verdüsung | entfällt | $5 \cdot 10^5$ Pa (5 bar) |
| Gaseintrittstemperatur | 150 °C | 148 °C |
| Gasaustrittstemperatur | 117 °C | 118 °C |
| Inertgasmenge (Kreisgas) | 30 t $N_2$ | 30 t $N_2$ |
| Pulverdaten | | |
| Schüttgewicht | 320 g/l | 80 g/l |
| Wassergehalt nach Karl Fischer | 1,6 % | 1,2 % |

**Patentansprüche**

1. Verfahren zur Herstellung von Alkylsulfat-Pulvern,
dadurch gekennzeichnet,
daß man die wäßrige Lösung oder Dispersion von Alkylsulfaten in den Kopfteil eines Sprühturmes durch Einstoffdüsen unter Druck versprüht und den so entstehenden Sprühnebel im Gleichstrom mit heißem Inertgas zu einem Pulver mit einem Schüttgewicht von mindestens 300 g/l trocknet.

2. Verfahren nach Anspruch 1,
dadurch gekennzeichnet,
daß der Vordruck der Flüssigkeit vor der Versprühung $10^6$ bis $10^7$ Pa beträgt.

3. Verfahren nach Anspruch 1,
dadurch gekennzeichnet,
daß die Alkylgruppe der Alkylsulfate linear oder verzweigt ist und 8 bis 22 Kohlenstoffatome enthält.

4. Verfahren nach Anspruch 1,
dadurch gekennzeichnet,
daß die wäßrige Lösung oder Dispersion 10 bis 60 Gew.-%, vorzugsweise 20 bis 50 Gew.-%, Alkylsulfate enthält.

5. Verfahren nach Anspruch 1,
   dadurch gekennzeichnet,
   daß als Inertgas Stickstoff verwendet wird.

6. Alkylsulfat-Pulver mit einem Schüttgewicht von mindestens 300 g/l hergestellt nach Anspruch 1.

**Europäisches Patentamt**

# EUROPÄISCHER RECHERCHENBERICHT

Nummer der Anmeldung

EP  93 11 1508

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | PATENT ABSTRACTS OF JAPAN vol. 1, no. 84 (C-77)1977 & JP-A-52 46 025 ( KAO  SEKKEN K.K. ) * Zusammenfassung * --- | 1 | C07C305/04 C07C303/24 C07C303/44 |
| A,D | DATABASE WPI Week 7939, Derwent Publications Ltd., London, GB; AN 79-70925B & JP-A-54 106 428 (LION FAT & OIL KK) * Zusammenfassung * ----- | 1 | |
| | | | **RECHERCHIERTE SACHGEBIETE (Int. Cl.5 )** |
| | | | C07C |

Der vorliegende Recherchenbericht wurde für alle Patentansprüche erstellt

| Recherchenort | Abschlußdatum der Recherche | Prüfer |
|---|---|---|
| BERLIN | 25 OKTOBER 1993 | KAPTEYN H. |